# EUROPÄISCHE PATENTSCHRIFT

(11) **EP 4 092 692 B1**
(45) Veröffentlichungstag und Bekanntmachung des Hinweises auf die Patenterteilung: **19.04.2023**
(21) Anmeldenummer: 22020165.1
(22) Anmeldetag: 12.04.2022
(51) Int. Cl.: H01C 1/14, A61B 5/00, A61N 1/04, A61B 5/024, H01R 4/00

(54) **VERFAHREN ZUR HERSTELLUNG EINER VERBINDUNG ZWISCHEN EINER ELEKTRODE UND EINEM ELEKTRISCHEN LEITER**
METHOD OF MAKING A CONNECTION BETWEEN AN ELECTRODE AND AN ELECTRICAL CONDUCTOR
PROCÉDÉ D'ÉTABLISSEMENT D'UNE CONNEXION ENTRE UNE ÉLECTRODE ET UN CONDUCTEUR ÉLECTRIQUE

(30) Priorität: 18.05.2021 DE 102021112839
(43) Veröffentlichungstag der Anmeldung: 23.11.2022
(73) Patentinhaber: NTT New Textile Technologies GmbH, 72336 Balingen (DE)
(72) Erfinder: Hans, Bauer, 72336 Balingen (DE)
(74) Vertreter: Müller, Gottfried

(56) Entgegenhaltungen:
- EP-A1- 0 521 181
- DE-A1-102006 016 861
- DE-A1-102008 042 554
- DE-T5-112016 002 798

## Beschreibung

Die Erfindung bezieht sich auf ein Verfahren zur Herstellung einer Verbindung zwischen einer Elektrode und einem elektrischen Leiter. Die Erfindung bezieht sich des Weiteren auf eine Elektrode, die mit einem elektrischen Leiter verbunden ist.

Bekannt sind Elektroden in Form von der Dehnungssensoren, die auf eine Stofflage aufgebracht sind, um die Dehnung der Stofflage zu überwachen. Beispielsweise wird in der DE 10 2008 042 554 A1 ein elastischer Brustgurt zur Überwachung der Atmung eines Patienten beschrieben, wobei auf den Brustgurt ein Dehnungssensor aufgenäht ist, der mit einer Auswerteeinheit auf dem Brustgurt verbunden ist.

Die JP 2014 025180 A offenbart eine Hose mit einem integrierten Dehnungssensor, der mithilfe eines elastischen Klebstoffes auf den Stoff der Hose aufgebracht ist. Über den Dehnungssensor können Dehnungen im Stoff der Hose registriert werden. Die Sensorsignale werden über einen ebenfalls in die Hose integrierten Sender auf ein externes Gerät übertragen.

Aus der EP 0 521 181 A1 ist ein PTC-Heizkörper mit einem Isolierrahmen bekannt, bei dem zwei auf beiden Seiten des Isolierrahmens angeordnete und mit dem Isolierrahmen jeweils an mindestens zwei Punkten fest verbundene Elektrodenplatten und mindestens ein in einem Durchbruch des Isolierrahmens eingesetztes, zwischen beiden Elektrodenplatten liegendes und mit diesen in Kontakt stehendes PTC-Element angeordnet sind. Die Befestigung jeder Elektrodenplatte in einem bestimmten Punkt am Isolierrahmen wird jeweils mittels einer integral an dem Isolierrahmen und der Elektrodenplatte ausgebildeten Steckverbindung erreicht.

Die DE 20 2004 004 182 U1 offenbart ein Textil, das zumindest teilweise elektrisch leitfähig ist und das über mindestens einen elektrischen Verbindungsleiter an eine elektrische Strom-/Spannungsquelle anschließbar ist, wobei der Verbindungsleiter an mindestens einer Lötstelle an das Textil gelötet ist.

Aus der EP 2 000 005 B1 ist die Herstellung eines Kontaktierungssystems zur elektrischen Kontaktierung von flächigen Heizelementen bekannt, wobei das Kontaktierungssystem einen schichtartigen Verbund mit einem Trägermaterial, einem metallischen Leiter und einer elektrisch leitfähigen Schicht aufweist. Der metallische Leiter wird auf das Trägermaterial aufgenäht. Anschließend wird der Verbund mit einem Lösungsmittel angefeuchtet, woraufhin die elektrisch leitfähige Schicht auf den Verbund auflaminiert wird.

Der Erfindung liegt die Aufgabe zugrunde, mit einfachen Maßnahmen eine sichere elektrische Verbindung zwischen einer Elektrode und einem elektrischen Leiter zu schaffen.

Diese Aufgabe wird erfindungsgemäß mit den Merkmalen der unabhängigen Ansprüche gelöst. Die abhängigen Ansprüche geben zweckmäßige Weiterbildungen an.

Die erfindungsgemäße Verbindung einer Elektrode mit einem elektrischen Leiter beziehungsweise das Verfahren zur Herstellung einer derartigen Verbindung kann beispielsweise für ein Bekleidungsstück, insbesondere für ein Unterbekleidungsstück oder gegebenenfalls auch für ein Oberbekleidungsstück eingesetzt werden. In Betracht kommt auch eine Verwendung für eine Bandage oder für einen Gurt. Eine Trägerlage, mit der die Elektrode verbunden werden kann, kann Bestandteil des Bekleidungsstücks, des Gurts bzw. der Bandage sein, oder es wird die Trägerlage mit dem Bekleidungsstück, dem Gurt oder der Bandage verbunden.

Mithilfe des erfindungsgemäßen Verfahrens wird eine Verbindung zwischen einer Elektrode und einem elektrischen Leiter hergestellt. In einem ersten Verfahrensschritt wird zunächst ein Leiterabschnitt des elektrischen Leiters durch eine Ausnehmung in einem Teil der Elektrode geführt. Eine Elektrodenzunge, die mit einer größeren Elektrodenfläche verbunden ist und über die Elektrodenfläche hinaussteht, wird in einem folgenden Verfahrensschritt in der Weise umgeklappt, dass der durch die Ausnehmung geführte Leiterabschnitt zwischen der Elektrodenzunge und der Elektrodenfläche liegt. Die Elektrodenzunge kann einteilig mit der Elektrodenfläche ausgebildet sein. Möglich ist auch eine separate Ausbildung von Elektrodenzunge und Elektrodenfläche, indem beispielsweise die Elektrodenzunge eine thermoadhäsive Folie und gegebenenfalls zusätzlich einen Klebefilm sowie optional eine Schutzlage umfasst.

Diese Ausführung hat den Vorteil, dass der Leiterabschnitt, welcher zur Kontaktierung mit der Elektrode dient, zwischen der Elektrodenzunge und der Elektrodenfläche an zwei Seiten anliegt, wodurch ein sicherer und dauerhafter elektrischer Kontakt gewährleistet ist. Der sichere Kontakt wird mit einer kompakten Bauform erreicht.

In einem weiteren, darauffolgenden Schritt kann die Elektrode einschließlich der Verbindung mit dem elektrischen Leiter auf eine Trägerlage aufgebracht und mit der Trägerlage verbunden werden. Die Trägerlage kann Bestandteil eines Kleidungsstücks, eines Gurts oder einer Bandage sein oder auf das Kleidungsstück, den Gurt oder die Bandage aufgebracht und verbunden werden. Die Trägerlage kann als eine Stofflage ausgebildet sein. Im Betracht kommen auch Ausführungen der Trägerlage aus einem anderen Material als Stoff, beispielsweise Ausführungen der Trägerlage als Metallgewebe.

Mit der Verbindung der Elektrode mit der Trägerlage ist auch gewährleistet, dass der zwischen der Elektrodenfläche und der Elektrodenzunge liegende Leiterabschnitt des elektrischen Leiters sicher und dauerhaft seine Position beibehält.

Es ist vorteilhaft, dass sich das Verfahren in einer einfachen Weise durchführen lässt. Bei dem Hindurchführen des Leiterabschnitts durch die Ausnehmung in der Elektrode und das Umklappen der Elektrodenzunge handelt es sich um mechanische Verfahrensschritte. Das Aufbringen der Elektrode auf die Trägerlage und das Verbinden der Elektrode mit der Trägerlage kann ebenfalls auf mechanische Weise oder gegebenenfalls auf thermische Weise oder eine Kombination von mechanischer und thermischer Weise durchgeführt werden.

Auf der Elektrodenzunge befindet sich ein Klebefilm, der gemeinsam mit der Elektrodenzunge umgeklappt wird. Ein Teil des Klebefilms ragt zwischen die Elektrodenfläche und eine Silikonschicht hinein, die sich auf der Elektrodenfläche befindet. Der Klebefilm, der beispielsweise aus einem thermoplastischen Polyurethan besteht, bildet einen Schutzfilm für die Elektrodenzunge. Der zwischen der Oberseite der Elektrodenfläche und der daraufliegenden Silikonschicht hineinragende Teil des Klebefilms kann die Elektrodenfläche entweder nur teilweise oder gegebenenfalls auch vollständig bedecken. Vorteilhafterweise wird der Klebefilm durch das Applizieren von Druck und Wärme, insbesondere durch Aufbügeln, auf die Elektrodenzunge und die Elektrodenfläche aufgebracht.

Gemäß einer weiteren vorteilhaften Ausführung ist der Leiter, der mit der Elektrodenzunge verbunden wird, von einem Klebetape abgedeckt, das zum Herstellen der Verbindung teilweise vom Leiter abgezogen wird, wobei der freiliegende Leiterabschnitt durch die Ausnehmung in der Elektrodenzunge geführt wird und anschließend das vom Leiter abgezogene Klebetape bis an die Kante der Elektrode angelegt wird. Diese Ausführung hat den Vorteil, dass das Klebetape des Leiters lückenlos an die Elektroden anschließt, so dass Schutz gegen mechanische Einwirkungen und gegen Wassereinwirkung gegeben ist.

Gemäß noch einer weiteren vorteilhaften Ausführung befindet sich auf der Unterseite der Elektrodenfläche eine thermoadhäsive Folie, die zum Verbinden der Elektrode mit der Trägerlage Druck und Wärme ausgesetzt wird. Unter dem Einfluss von Druck und Wärme schmilzt die thermoadhäsive Folie an, so dass das Material der thermoadhäsiven Folie zumindest teilweise in die Trägerlage eindringen kann. Nach dem Aushärten ist die thermoadhäsive Folie und damit auch die Elektrode fest mit der Trägerlage verbunden. Das Aufbringen von Druck und Wärme kann beispielsweise mittels Bügeln durchgeführt werden.

Gemäß einer weiteren vorteilhaften Ausführung wird die thermoadhäsive Folie auf der Unterseite der Elektrodenfläche gemeinsam mit der Elektrodenzunge umgeklappt, insbesondere um 180°. Aufeinanderliegende Abschnitte der thermoadhäsiven Folie werden anschließend durch Druck und Wärme verbunden, insbesondere mittels Bügeln.

In die thermoadhäsive Folie können hintereinanderliegend Durchbrechungen eingebracht sein, die im umgeklappten Zustand der Elektrodenzunge unmittelbar aufeinanderliegen. Im Bereich der Durchbrechungen befindet sich der Leiterabschnitt, der durch die Durchbrechungen hindurchragt und auf Kontakt zu der dahinterliegenden Elektrodenfläche liegt. Auf diese Weise ist ein unmittelbarer Kontakt des Leiterabschnittes mit der Elektrodenfläche gewährleistet.

Gemäß einer weiteren vorteilhaften Ausführung sind der Klebefilm und die thermoadhäsive Folie auf unterschiedliche Seiten der Elektrodenzunge aufgebracht und können gemeinsam mit der Elektrodenzunge umgeklappt werden. Der Klebefilm schützt die Elektrodenzunge, ein Abschnitt des Klebefilms liegt außerdem auf der Elektrodenfläche. Über die thermoadhäsive Folie wird die Elektrode mit der Trägerlage verbunden.

Die Elektrodenfläche ist, gemäß weiterer zweckmäßiger Ausführung, von einer Silikonschicht bedeckt, die nach dem Aufbringen der Elektrode auf die Trägerlage erwärmt wird, wobei das Silikon der Silikonschicht sich mit der Trägerlage verbindet. Das Erwärmen kann ebenfalls durch Druck und Wärme erfolgen und beispielsweise im Wege des Aufbügelns durchgeführt werden.

Gemäß noch einer weiteren vorteilhaften Ausführung ist auf die Unterseite der Elektrode ein Schutzfilm bzw. eine Schutzlage aufgebracht, der bzw. die vor dem Aufbringen der Elektrode auf die Trägerlage und dem Verbinden mit der Trägerlage entfernt wird.

Die Erfindung bezieht sich des Weiteren auf eine Elektrode, die gemäß des vorbeschriebenen Verfahrens hergestellt sein kann, gegebenenfalls aber auch auf eine andere Verfahrensweise hergestellt sein kann. Der Elektrode ist wie vorbeschrieben ein elektrischer Leiter zugeordnet, der mit der Elektrode verbunden ist, wobei ein Leiterabschnitt des Leiters durch eine Ausnehmung in der Elektrode geführt ist. Eine Elektrodenzunge, die Teil der Elektrode ist und über eine Elektrodenfläche hinaussteht, ist in der Weise umgeklappt, insbesondere um 180°, dass der durch die Ausnehmung geführte Leiterabschnitt zwischen der Elektrodenzunge und der Elektrodenfläche liegt. Die Elektrodenzunge kann einteilig mit der Elektrodenfläche oder separat von der Elektrodenfläche ausgebildet, jedoch über eine weitere Lage bzw. Schicht der Elektrode mit der Elektrodenfläche verbunden sein.

Die Elektrodenfläche kann als elektrisch leitfähige Textillage ausgebildet und die Elektrodenzunge einteilig mit der Textillage ausgeführt sein.

Die Elektrodenzunge kann streifen- oder rechteckförmig ausgebildet sein oder, in einer weiteren Ausführung, über einen schmalen Zungenabschnitt mit einer Lage bzw. Schicht oder mehreren Lagen bzw. Schichten der Elektrode verbunden sein und einen sich an den schmalen Zungenabschnitt verbreiterten Leiterbefestigungsabschnitt aufweisen, über den der elektrische Leiter im umgeklappten Zustand der Elektrodenzunge an der Elektrode befestigt ist.

Die Elektrode kann mit einer Trägerlage verbunden sein. Eine in dieser Weise ausgeführte Kombination einer Trägerlage und einer Elektrode lässt sich in einfacher Weise herstellen und zeichnet sich durch eine dauerhafte und sichere Verbindung des elektrischen Leiters mit der Elektrode aus.

Die Elektrode umfasst vorteilhafterweise eine Silikonschicht, die auf die Elektrodenfläche aufgebracht ist oder in die die Elektrodenfläche eingebettet ist. Die Silikonschicht ist insbesondere elektrisch leitfähig ausgebildet, beispielsweise durch das Einbringen von elektrisch leitfähigen Partikeln, insbesondere Silberpartikeln oder Carbon in Form von Partikeln oder Fasern.

Gemäß noch einer weiteren vorteilhaften Ausführung besteht der auf die Elektrodenzunge aufgebrachte Klebefilm aus einem thermoplastischen Polyurethan.

Elektrodenfläche und Elektrodenzunge können unabhängig von dem Herstellungsverfahren als eine elektrisch leitfähige Textillage ausgeführt sein. Die Leitfähigkeit wird beispielsweise durch Silberpartikeln oder Carbon in Form von Partikeln oder Fasern in der Textillage erreicht.

Die Elektrode, insbesondere die Elektrodenfläche, bildet einen Sensor, der insbesondere zur Erfassung von Vitalparametern ausgebildet ist, beispielsweise als Pulssensor zur Pulserfassung. Über die Elektrode als Sensor können gegebenenfalls elektrische Spannungsänderungen auf der Haut erfasst werden. Die Elektrode kann in einer weiteren Ausführung auch zur Muskelstimulation durch Strompulse verwendet werden.

Es ist auch möglich, die Elektrode als einen Dehnungssensor auszubilden, der resistiv ausgeführt ist, so dass sich der elektrische Widerstand des Dehnungssensors mit der Dehnung der Elektrode ändert.

Desweiteren ist es möglich, eine kapazitiv ausgebildete Elektrodenfläche als Sensor zu verwenden, deren elektrische Kapazität sich bei einer Dehnung ändert.

Weitere Vorteile und zweckmäßige Ausführungen sind den weiteren Ansprüchen, der Figurenbeschreibung und den Zeichnungen zu entnehmen. Es zeigen:
- Figur 1: eine perspektivische Ansicht einer Elektrode, die aus verschiedenen Schichten aufgebaut ist und sich zur Verwendung auf einer Stofflage eignet, die beispielsweise Bestandteil eines Bekleidungsstücks, einer Bandage oder eines Gurts ist,
- Figur 2: eine Draufsicht auf eine Elektrode,
- Figur 3: eine Draufsicht auf eine schmaler ausgebildete Elektrode,
- Figur 4a: in perspektivischer Ansicht eine Elektrode während eines ersten Verfahrensschrittes zur Verbindung mit einem elektrischen Leiter,
- Figur 4b: eine Ausschnittvergrößerung aus Figur 4a,
- Figur 5a: eine weitere perspektivische Ansicht der Elektrode während eines zweiten Verfahrensschrittes zur Verbindung mit dem elektrischen Leiter,
- Figur 5b: eine Ausschnittvergrößerung aus Figur 5a,
- Figur 6: die mit einer Stofflage verbundene Elektrode,
- Figur 7a: eine Draufsicht auf eine Elektrode in einer weiteren Ausführung,
- Figur 7b: die Elektrode aus Fig. 7a in einer Ansicht von unten,
- Figur 8a: eine Draufsicht auf eine Elektrode in noch einer weiteren Ausführung,
- Figur 8b: die Elektrode aus Fig. 8a in einer Ansicht von unten.

In den Figuren sind gleiche Bauteile mit gleichen Bezugszeichen versehen.

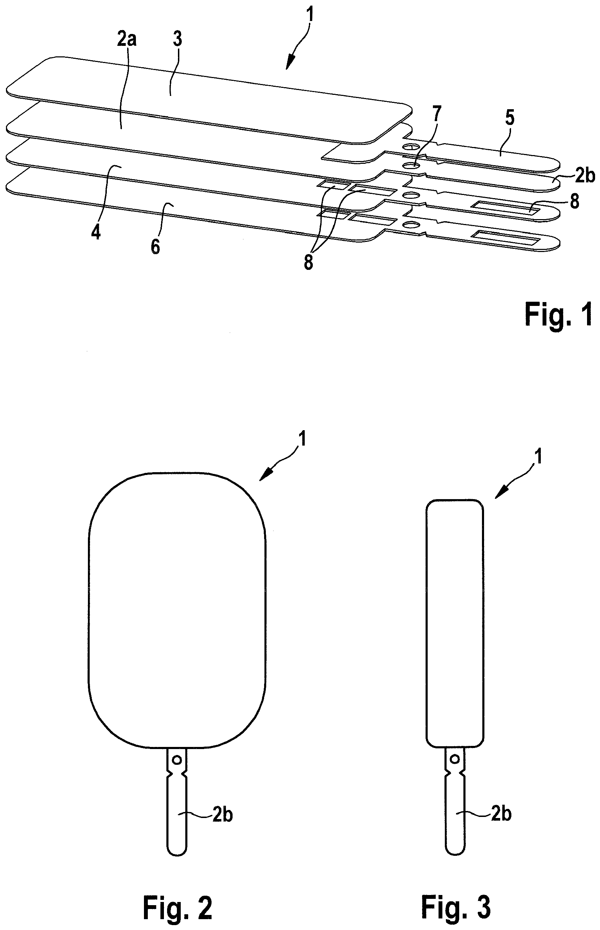

In Figur 1 ist der schichtweise Aufbau einer Elektrode 1 dargestellt, die als Sensor in Trägerlagen verwendet werden kann, beispielsweise zur Erfassung von Vitalparametern, zur Muskelstimulation oder zur Feststellung der Dehnung der Trägerlage in einer Ausführung als Stofflage. Die Elektrode 1 umfasst eine Elektrodenfläche 2a, die als elektrisch leitfähige Textillage ausgeführt ist, und einen einteilig mit der Elektrodenfläche 2a ausgebildete Elektrodenzunge 2b. Die Elektrodenfläche 2a ist rechteckförmig ausgebildet, wobei sich die Elektrodenzunge 2b an eine schmale Stirnseite der Elektrodenfläche 2a anschließt.

Die Elektrode 1 umfasst des Weiteren eine elektrisch leitende Silikonschicht 3, die auf die Oberseite der Elektrodenfläche 2a aufgebracht ist. Die elektrische Leitfähigkeit wird durch Einbringen von elektrisch leitenden Partikeln, wie zum Beispiel Silberpartikeln oder Carbon in Form von Partikeln oder Fasern in das Silikon der Silikonschicht 3 erreicht.

Auf der Unterseite der Elektrodenfläche 2a - der der Silikonschicht 3 gegenüberliegenden Seite - befindet sich eine thermoadhäsive Folie 4, die zum Verbinden der Elektrode 1 mit einer Stofflage verwendet wird. Die Schichten 2a, 3 und 4 bilden eine zusammengefügte, miteinander verbundene Einheit. Teil der Elektrode ist außerdem ein Klebefilm 5, der auf der Oberseite der Elektrodenzunge 2b aufgebracht ist, wobei ein Teil des Klebefilms 5 zwischen die Elektrodenfläche 2a und die darüber liegende Silikonschicht 3 hineinragt. Die thermoadhäsive Folie 4 deckt die Oberseite der Elektrodenzunge 2b ab und bietet einen Schutz für die Elektrodenzunge 2b gegen mechanische Einwirkungen und gegen Feuchtigkeit.

An der Unterseite der thermoadhäsiven Folie 4 befindet sich eine Schutzlage 6, die als ein Trägerpapier ausgebildet ist und vor dem Aufbringen der Elektrode 1 auf eine Stofflage abgezogen wird.

Sowohl die thermoadhäsive Folie 4 als auch die Schutzlage 6 erstrecken sich sowohl über die Elektrodenfläche 2a als auch über die Elektrodenzunge 2B.

In die Elektrodenzunge 2b ist eine kreisförmige Ausnehmung 7 eingebracht. Entsprechende Ausnehmungen befindet sich auch in dem darüber liegenden Klebefilm 5 sowie in der darunterliegenden thermoadhäsiven Folie 4. Die Ausnehmungen in dem Klebefilm 5 und der Folie 4 fluchten mit der Ausnehmung 7.

In die thermoadhäsive Folie 4 sind mehrere Durchbrechungen 8 eingebracht. Eine erste Durchbrechung 8 befindet sich am zungenförmigen Fortsatz der Folie 4, weitere Durchbrechungen befinden sich an einer vergrößerten Fläche der Folie 4, die mit der Elektrodenfläche 2a korrespondiert. Bei einem Umklappen der Elektrodenzunge 2b nach unten werden auch der darüber liegende Klebefilm 5 sowie der zungenförmige Fortsatz der darunterliegenden Folie 4 mit umgeklappt. Bei diesem Umklappvorgang gelangen die Durchbrechungen 8 in der Folie 4 in Überdeckung.

Die Figuren 2 und 3 zeigen verschiedene Ausführungsvarianten der Elektrode 1 mit unterschiedlichen Breiten. Je nach Ausführung kann die Elektrode 1 eine größere Breite (Figur 2) oder eine geringere Breite (Figur 3) aufweisen. In jedem Fall ist die Elektrode 1 mit einer Elektrodenzunge 2B versehen, die zur Anbindung eines elektrischen Leiters an die Elektrode dient.

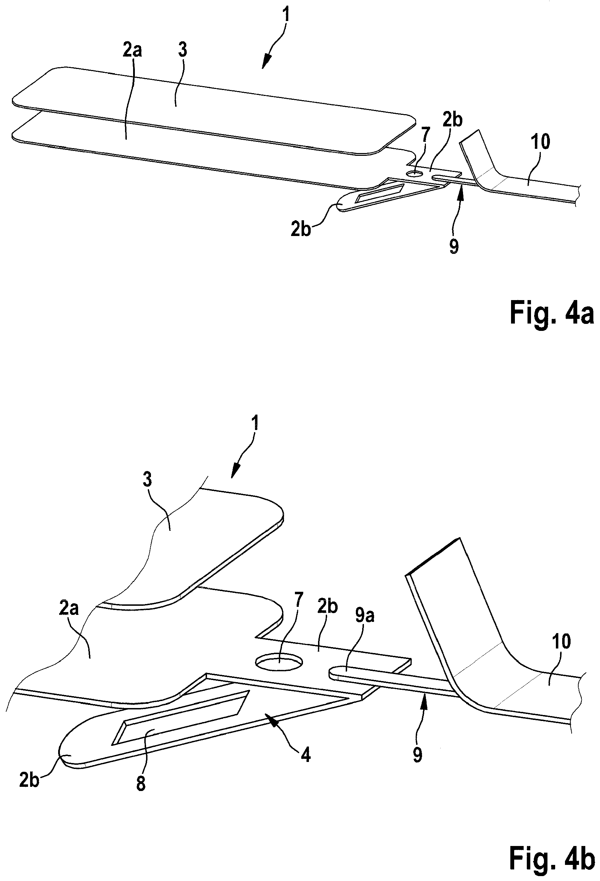

In Figur 4a und der vergrößerten Darstellung gemäß Figur 4b ist ein erster Verfahrensschritt zur Verbindung der Elektrode mit einem elektrischen Leiter 9 dargestellt. Die Elektrodenzunge 2b wird im Anschluss an die Ausnehmung 7 nach unten geklappt, und der elektrische Leiter 9 wird mit seinem stirnseitigen Leiterabschnitt 9a durch die Ausnehmung 7 in der Elektrodenzunge 2b hindurchgeführt. Zu erkennen ist in Figur 4a und 4b auch die Durchbrechung 8, die in die thermoadhäsive Folie 4 eingebracht ist, welche sich auf der Unterseite der Elektrodenzunge 2b befindet.

Der elektrische Leiter 9 ist von einem Klebetape 10 überdeckt, das an einem Ende angehoben wird, um den stirnseitigen Abschnitt 9a des Leiters 9 freizulegen.

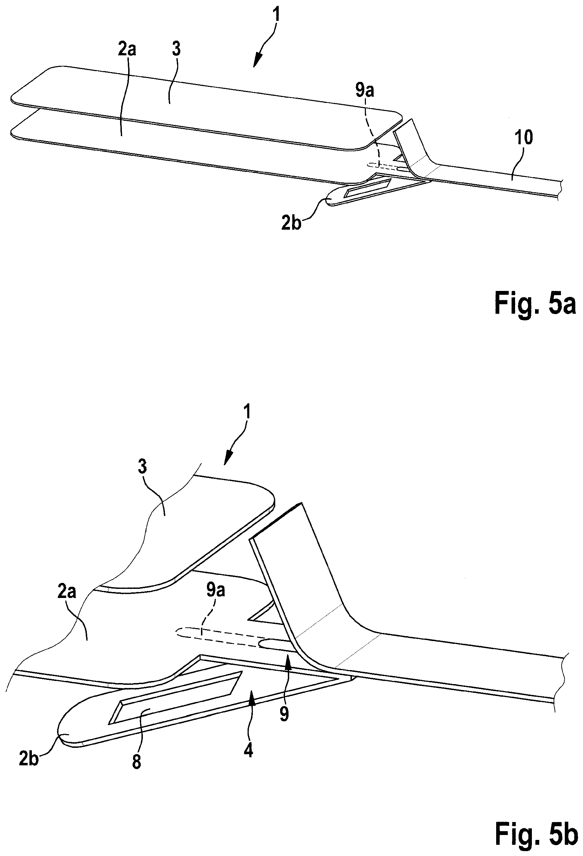

In den Figuren 5a und 5b ist der stirnseitige Leiterabschnitt 9a des elektrischen Leiters 9 bereits durch die Ausnehmung in der Elektrodenzunge 2b hindurchgeführt und befindet sich nun unterhalb der Elektrodenzunge 2b und teilweise auch unterhalb der Elektrodenfläche 2a. Der unterhalb liegende Leiterabschnitt 9a kommt bei der vollständig umgeklappten Elektrodenzunge 2b in der Durchbrechung 8 der thermoadhäsiven Folie 4 zu liegen, so dass der Leiterabschnitt 9a nur unmittelbar in Kontakt gelangt mit dem umgeklappten Teil der Elektrodenzunge 2b.

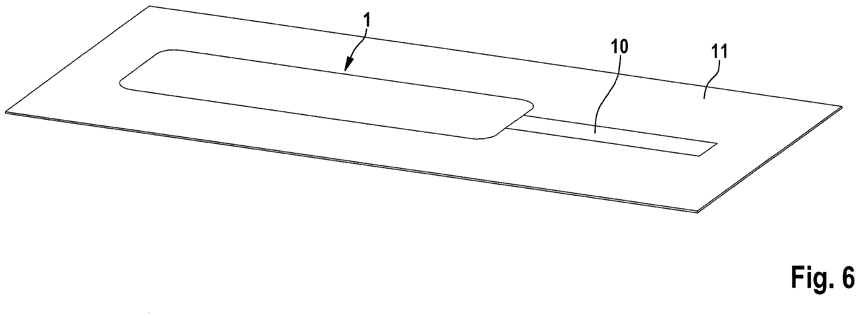

Der elektrische Leiter 9 wird mit seinem Leiterabschnitt 9a so weit in die Ausnehmung in der Elektrodenzunge 2b hineingeschoben, dass die vordere Stirnkante des Klebetapes 10 im heruntergeklappten Zustand an der zugewandten Stirnkante der Elektrode 1 anliegt. Dieser Zustand ist in Figur 6 dargestellt. Diese Ausführung hat den Vorteil, dass das Klebetape 10 bündig an die Elektrode 1 anschließt.

In Figur 6 ist die Elektrode 1 mit einer Stofflage 11 verbunden. Die Verbindung zwischen der Elektrode 1 und der Stofflage 11 erfolgt über die thermoadhäsive Folie 4 auf der Unterseite der Elektrodenfläche 2a und der Elektrodenzunge 2b. Hierfür wird Druck und Wärme aufgebracht, beispielsweise durch Aufbügeln, wodurch sich die thermoadhäsive Folie 4 erwärmt und eine Verbindung mit der darunterliegenden Stofflage 11 eingeht, indem angeschmolzenes Material der Folie 4 in die Poren der Stofflage 11 eindringt und dort aushärtet. Der zungenartige Vorsprung der thermoadhäsiven Folie 4, welcher der Elektrodenzunge 2b entspricht, gelangt im umgeklappten Zustand in Kontakt mit dem größeren Abschnitt der Folie 4 und wird unmittelbar mit diesem größeren Abschnitt verbunden.

Beim Aufbügeln wird außerdem die obenliegende Silikonschicht 3 erwärmt, so dass auch diese Schicht 3 anschmelzen kann und sich seitlich ausbreitendes Silikonmaterial in Kontakt mit der Stofflage 11 gelangt. Nach dem Auskühlen und Aushärten besteht auch eine Verbindung zwischen der Silikonschicht 3 und der Stofflage 11.

Der elektrische Leiter 9 kann zu einer Auswerte- und Anzeigeeinheit geführt werden, in der elektrische Signale der Elektrode 1 ausgewertet und angezeigt werden. Die Auswerte- und Anzeigeeinheit kann sich ebenfalls an der Stofflage befinden.

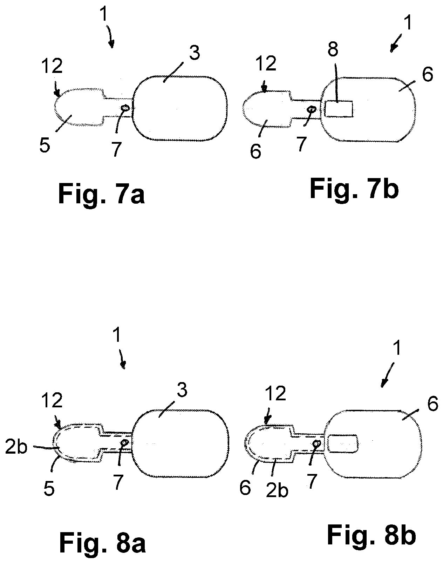

In den Figuren 7a und 7b ist eine Elektrode 1 in einer weiteren Ausführung in Draufsicht und in einer Ansicht von unten dargestellt, die grundsätzlich die gleichen Lagen bzw. Schichten wie in den vorangegangenen Beispielen aufweist mit einer obenliegenden Silikonschicht 3, einer darunterliegenden Elektrodenfläche, welche vorzugsweise als elektrisch leitfähige Textillage ausgebildet ist, einer darunterliegenden thermoadhäsiven Folie und einer untenliegenden Schutzlage 6. Außerdem ragt ein Klebefilm 5 zwischen die Silikonschicht 3 und die Elektrodenfläche hinein.

Der Klebefilm 5 ist Bestandteil der Elektrodenzunge 12, die außerdem von einem Teil der thermoadhäsiven Folie und der Schutzlage 6 gebildet ist, nicht jedoch von der Elektrodenfläche, welche sich nur über den Hauptbestandteil der Elektrode 1 erstreckt. Dementsprechend kann Material der Elektrodenfläche eingespart werden.

Die verschiedenen Schichten sind im Bereich der Elektrodenzunge 12 mit einer Durchbrechung 8 für den elektrischen Leiter versehen. Außerdem sind die Schutzlage 6 und die thermoadhäsive Folie mit einer sich deckenden Durchbrechung 8 versehen, in die im umgeklappten Zustand der elektrische Leiter hineinragt, um in Kontakt mit der Elektrodenfläche zu gelangen.

Die Elektrodenzunge 12 ist über einen schmalen Zungenabschnitt mit einer Lage der Elektrode verbunden und weist einen sich an den schmalen Zungenabschnitt anschließenden, verbreiterten Leiterbefestigungsabschnitt auf, über den der elektrische Leiter im umgeklappten Zustand der Elektrodenzunge 12 an der Elektrode 1 befestigt ist.

In den Figuren 8a und 8b ist eine Elektrode 1 in Draufsicht und in einer Ansicht von unten dargestellt, die weitgehend dem vorhergehenden Ausführungsbeispiel gemäß den Figuren 7a und 7b entspricht, jedoch mit dem Unterschied, dass bei Fig. 8a und 8b sich die Elektrodenfläche in Form der einteilig mit der Elektrodenfläche ausgebildeten Zunge 2b bis zu der Elektrodenzunge 12 erstreckt. Die gestrichelt dargestellte Zunge 2b ist nach oben von dem Klebefilm 5 und nach unten von der thermoadhäsiven Folie und der Schutzlage 6 bedeckt. Die Zunge 2b ist geringfügig kleiner als die weiteren Schichten im Bereich der Elektrodenzunge 12 und wird daher auch im Randbereich vollständig von den darüber und darunter liegenden Schichten abgedeckt, wodurch das Material der Zunge 2b vor äußeren Einflüssen geschützt ist.

## Patentansprüche

1. Verfahren zur Herstellung einer Verbindung zwischen einer Elektrode (1) und einem elektrischen Leiter (9), mit den folgenden Verfahrensschritten:
- Hindurchführen eines Leiterabschnitts (9a) des Leiters (9) durch eine Ausnehmung (7) in einem Teil der Elektrode (1),
- Umklappen einer Elektrodenzunge (2b), die
mit einer Elektrodenfläche (2a) verbunden ist und über die Elektrodenfläche (2a) hinaussteht, in der Weise, dass der durch die Ausnehmung (7) geführte Leiterabschnitt (9a) zwischen der Elektrodenzunge (2b) und der Elektrodenfläche (2a) liegt,
**dadurch gekennzeichnet,**
**dass** ein auf die Elektrodenzunge (2b) aufgebrachter Klebefilm (5), der zwischen die Elektrodenfläche (2a) und eine Silikonschicht (3) auf der Elektrodenfläche (2a) hineinragt, gemeinsam mit der Elektrodenzunge (2b) umgeklappt wird.

2. Verfahren nach Anspruch 1,
**dadurch gekennzeichnet,**
**dass** der Leiter (9) von einem Klebetape (10) abgedeckt ist, das teilweise vom Leiter (9) abgezogen wird, wobei der freiliegende Leiterabschnitt (9a) durch die Ausnehmung (7) in der Elektrodenzunge (2b) geführt wird und anschließend das vom Leiter (9) abgezogene Klebetape (10) bis an die Kante der Elektrode (1) angelegt wird.

3. Verfahren nach Anspruch 1 oder 2,
**dadurch gekennzeichnet,**
**dass** die Elektrode (1) auf eine Trägerlage (11) aufgesetzt und die Elektrode (1) mit der Trägerlage (11) verbunden wird.

4. Verfahren nach Anspruch 3,
**dadurch gekennzeichnet,**
**dass** zum Verbinden der Elektrode (1) mit der Trägerlage (11) eine thermoadhäsive Folie (4) auf der Unterseite der Elektrodenfläche (2a) Druck und Wärme ausgesetzt wird.

5. Verfahren nach einem der Ansprüche 1 bis 4,
**dadurch gekennzeichnet,**
**dass** eine thermoadhäsive Folie (4) auf der Unterseite der Elektrodenfläche (2a) gemeinsam mit der Elektrodenzunge (2b) umgeklappt wird, wobei aufeinanderliegende Abschnitte der thermoadhäsiven Folie (4) durch Druck und Wärme verbunden werden.

6. Verfahren nach Anspruch 5,
**dadurch gekennzeichnet,**
**dass** in die aufeinanderliegenden Abschnitte Durchbrechungen (8) in die thermoadhäsive Folie (4) eingebracht sind, in denen der Leiter (9) auf Kontakt zur Elektrodenfläche (2a) liegt.

7. Verfahren nach Anspruch 1 und einem der Ansprüche 4 bis 6,
**dadurch gekennzeichnet,**
**dass** der Klebefilm (5) und die thermoadhäsive Folie (4) auf unterschiedliche Seiten der Elektrodenzunge (2b) aufgebracht sind und gemeinsam mit der Elektrodenzunge (2b) umgeklappt werden.

8. Verfahren nach einem der Ansprüche 1 bis 7,
**dadurch gekennzeichnet,**
**dass** eine die Elektrodenfläche (2a) bedeckende Silikonschicht (3) nach dem Aufbringen der Elektrode (1) auf eine Trägerlage (11) erwärmt wird, wodurch sich das Silikon der Silikonschicht (3) mit der Trägerlage (11) verbindet.

9. Verfahren nach einem der Ansprüche 1 bis 8,
**dadurch gekennzeichnet,**
**dass** auf die Unterseite der Elektrode (1) eine Schutzlage (6) aufgebracht ist, die vor dem Aufbringen der Elektrode (1) auf die Trägerlage (11) und Verbinden der Elektrode (1) mit der Trägerlage (11) entfernt wird.

10. Elektrode-Leiter-Kombination, insbesondere hergestellt nach dem Verfahren nach einem der Ansprüche 1 bis 9, mit einer Elektrode (1) und einem mit der Elektrode (1) verbundenen elektrischen Leiter (9), wobei ein Leiterabschnitt (9a) des Leiters (9) durch eine Ausnehmung (7) in einem Teil der Elektrode (1) geführt ist, wobei eine Elektrodenzunge (2b), die mit einer Elektrodenfläche (2a) verbunden ist und über die Elektrodenfläche (2a) hinaussteht, in der Weise umgeklappt ist, dass der durch die Ausnehmung (7) geführte Leiterabschnitt (9a) zwischen der Elektrodenzunge (2b) und der Elektrodenfläche (2a) liegt, wobei auf die Elektrodenzunge (2b) ein Klebefilm (5) aufgebracht ist, der gemeinsam mit der Elektrodenzunge (2b) umgeklappt wird.

11. Elektrode-Leiter-Kombination nach Anspruch 10,
**dadurch gekennzeichnet,**
**dass** die Elektrodenfläche (2a) als elektrisch leitfähige Textillage ausgebildet und die Elektrodenzunge (2b) einteilig mit der Textillage ausgeführt ist.

12. Elektrode-Leiter-Kombination nach Anspruch 10 oder 11,
**dadurch gekennzeichnet,**
**dass** die Elektrode (1) eine Silikonschicht (3) umfasst, die auf die Elektrodenfläche (2a) aufgebracht ist oder in die die Elektrodenfläche (2a) eingebettet ist.

13. Elektrode-Leiter-Kombination nach einem der Ansprüche 10 bis 12,
**dadurch gekennzeichnet,**
**dass** der auf die Elektrodenzunge (2b) aufgebrachte Klebefilm (5) aus einem thermoplastischen Polyurethan besteht.

14. Trägerlage-Elektrode-Leiter-Kombination mit einer Elektrode-leiter-Kombination nach einem der Ansprüche 10 bis 13,
**dadurch gekennzeichnet,**
**dass** die Elektrode (1) mit einer Trägerlage (11) verbunden ist.

15. Bekleidungsstück mit einer Elektrode-Leiter-Kombination nach einem der Ansprüche 10 bis 13.

16. Bandage mit einer Elektrode-Leiter-Kombination nach einem der Ansprüche 10 bis 13.

17. Gurt mit einer Elektrode-Leiter-Kombination nach einem der Ansprüche 10 bis 13.

## Claims

1. Method for producing a connection between an electrode (1) and an electrical conductor (9), comprising the following method steps:
- guiding a conductor section (9a) of the conductor (9) through a cutout (7) in a part of the electrode (1),
- folding over an electrode tab (2b) which is connected to an electrode surface (2a) and projects beyond the electrode surface (2a), in such a way that the conductor section (9a) guided through the cutout (7) lies between the electrode tab (2b) and the electrode surface (2a),
**characterized**
**in that** an adhesive film (5) applied to the electrode tab (2b) and projecting in between the electrode surface (2a) and a silicone layer (3) on the electrode surface (2a) is folded over jointly with the electrode tab (2b).

2. Method according to Claim 1,
**characterized**
**in that** the conductor (9) is covered by an adhesive tape (10), which is partly pulled off the conductor (9), wherein the exposed conductor section (9a) is guided through the cutout (7) in the electrode tab (2b) and subsequently the adhesive tape (10) pulled off the conductor (9) is placed right up to the edge of the electrode (1).

3. Method according to Claim 1 or 2,
**characterized**
**in that** the electrode (1) is placed onto a carrier ply (11) and the electrode (1) is connected to the carrier ply (11).

4. Method according to Claim 3,
**characterized**
**in that** for the purpose of connecting the electrode (1) to the carrier ply (11), a thermoadhesive film (4) on the underside of the electrode surface (2a) is subjected to pressure and heat.

5. Method according to any of Claims 1 to 4,
**characterized**
**in that** a thermoadhesive film (4) on the underside of the electrode surface (2a) is folded over jointly with the electrode tab (2b) wherein sections of the thermoadhesive film (4) that lie one on top of another are connected by pressure and heat.

6. Method according to Claim 5,
**characterized**
**in that** perforations (8) into the thermoadhesive film (4) are introduced into the sections that lie one on top of another, in which perforations the conductor (9) is in contact with the electrode surface (2a).

7. Method according to Claim 1 and any of Claims 4 to 6,
**characterized**
**in that** the adhesive film (5) and the thermoadhesive film (4) are applied to different sides of the electrode tab (2b) and are folded over jointly with the electrode tab (2b).

8. Method according to any of Claims 1 to 7,
**characterized**
**in that** a silicone layer (3) covering the electrode surface (2a) is heated after the electrode (1) has been applied to a carrier ply (11), as a result of which the silicone of the silicone layer (3) bonds to the carrier ply (11).

9. Method according to any of Claims 1 to 8,
**characterized**
**in that** a protective ply (6) is applied to the underside of the electrode (1), which protective ply is removed before the process of applying the electrode (1) to the carrier ply (11) and connecting the electrode (1) to the carrier ply (11).

10. Electrode-conductor combination, in particular produced according to the method according to any of Claims 1 to 9, comprising an electrode (1) and an electrical conductor (9) connected to the electrode (1), wherein a conductor section (9a) of the conductor (9) is guided through a cutout (7) in a part of the electrode (1), wherein an electrode tab (2b), which is connected to an electrode surface (2a) and projects beyond the electrode surface (2a) is folded over in such a way that the conductor section (9a) guided through the cutout (7) lies between the electrode tab (2b) and the electrode surface (2a), wherein an adhesive film (5) is applied to the electrode tab (2b), which adhesive film is folded over jointly with the electrode tab (2b) .

11. Electrode-conductor combination according to Claim 10,
**characterized**
**in that** the electrode surface (2a) is configured as an electrically conductive textile ply and the electrode tab (2b) is embodied integrally with the textile ply.

12. Electrode-conductor combination according to Claim 10 or 11,
**characterized**
**in that** the electrode (1) comprises a silicone layer (3), which is applied to the electrode surface (2a) or into which the electrode surface (2a) is embedded.

13. Electrode-conductor combination according to any of Claims 10 to 12,
**characterized**
**in that** the adhesive film (5) applied to the electrode tab (2b) consists of a thermoplastic polyurethane.

14. Carrier ply-electrode-conductor combination comprising an electrode-conductor combination according to any of Claims 10 to 13,
**characterized**
**in that** the electrode (1) is connected to a carrier ply (11).

15. Garment comprising an electrode-conductor combination according to any of Claims 10 to 13.

16. Bandage comprising an electrode-conductor combination according to any of Claims 10 to 13.

17. Strap comprising an electrode-conductor combination according to any of Claims 10 to 13.

## Revendications

1. Procédé d'établissement d'une liaison entre une électrode (1) et un conducteur électrique (9), ledit procédé comprenant les étapes suivantes :
- faire passer une portion de conducteur (9a) du conducteur (9) à travers un évidement (7) ménagé dans une partie de l'électrode (1),
- replier une languette d'électrode (2b) qui est reliée à une surface d'électrode (2a) et qui fait saillie de la surface d'électrode (2a) de manière à ce que la portion de conducteur (9a) guidée à travers l'évidement (7) soit située entre la languette d'électrode (2b) et la surface d'électrode (2a),
**caractérisé en ce que**
un film adhésif (5) appliqué sur la languette d'électrode (2b) et faisant saillie entre la surface d'électrode (2a) et une couche de silicone (3) sur la surface d'électrode (2a) est replié avec la languette d'électrode (2b).

2. Procédé selon la revendication 1,
**caractérisé en ce que**
le conducteur (9) est recouvert d'un ruban adhésif (10) qui est partiellement retiré du conducteur (9), la portion de conducteur exposée (9a) étant guidée à travers l'évidement (7) ménagé dans la languette d'électrode (2b) puis le ruban adhésif (10) retiré du conducteur (9) étant appliqué jusqu'au bord de l'électrode (1).

3. Procédé selon la revendication 1 ou 2,
**caractérisé en ce que**
l'électrode (1) est placée sur une couche de support (11) et l'électrode (1) est reliée à la couche de support (11).

4. Procédé selon la revendication 3,
**caractérisé en ce que**
pour relier l'électrode (1) à la couche de support (11), une pellicule thermo-adhésive (4) placée sur le côté inférieur de la surface d'électrode (2a) est exposée à la pression et à la chaleur.

5. Procédé selon l'une des revendications 1 à 4,
**caractérisé en ce que**
une pellicule thermo-adhésive (4) est repliée sur le côté inférieur de la surface d'électrode (2a) conjointement avec la languette d'électrode (2b), des portions superposées de la pellicule thermo-adhésive (4) étant reliées par pression et chaleur.

6. Procédé selon la revendication 5,
**caractérisé en ce que**
des ouvertures (8), dans lesquelles le conducteur (9) est en contact avec la surface d'électrode (2a), sont pratiquées dans la pellicule thermo-adhésive (4) dans les portions superposées.

7. Procédé selon la revendication 1 et l'une des revendications 4 à 6,
**caractérisé en ce que**
le film adhésif (5) et la pellicule thermo-adhésive (4) sont appliqués sur des côtés différents de la languette d'électrode (2b) et sont repliés conjointement avec la languette d'électrode (2b).

8. Procédé selon l'une des revendications 1 à 7,
**caractérisé en ce que**
une couche de silicone (3) recouvrant la surface d'électrode (2a) est chauffée après que l'électrode (1) a été appliquée sur une couche de support (11) de façon à lier le silicone de la couche de silicone (3) à la couche de support (11).

9. Procédé selon l'une des revendications 1 à 8,
**caractérisé en ce que**
une couche de protection (6), qui est retirée avant que l'électrode (1) ne soit appliquée sur la couche de support (11) et que l'électrode (1) ne soit reliée à la couche de support (11), est appliquée sur le côté inférieur de l'électrode (1).

10. Ensemble électrode-conducteur, notamment réalisé par le procédé selon l'une des revendications 1 à 9, comprenant une électrode (1) et un conducteur électrique (9) relié à l'électrode (1), une portion de conducteur (9a) du conducteur (9) étant guidée à travers un évidement (7) ménagé dans une partie de l'électrode (1), une languette d'électrode (2b), qui est reliée à une surface d'électrode (2a) et qui fait saillie de la surface d'électrode (2a), étant repliée de manière à ce que la portion de conducteur (9a) guidée à travers l'évidement (7) soit située entre la languette d'électrode (2b) et la surface d'électrode (2a), un film adhésif (5) étant appliqué sur la languette d'électrode (2b) et étant replié conjointement avec la languette d'électrode (2b).

11. Ensemble électrode-conducteur selon la revendication 10,
**caractérisé en ce que**
la surface d'électrode (2a) est conçue sous la forme d'une couche textile électriquement conductrice et la languette d'électrode (2b) est réalisée d'une seule pièce avec la couche textile.

12. Ensemble électrode-conducteur selon la revendication 10 ou 11,
**caractérisé en ce que**
l'électrode (1) comprend une couche de silicone (3) qui est appliquée sur la surface d'électrode (2a) ou dans laquelle la surface d'électrode (2a) est incorporée.

13. Ensemble électrode-conducteur selon l'une des revendications 10 à 12,
**caractérisé en ce que**
le film adhésif (5) appliqué sur la languette d'électrode (2b) est en polyuréthane thermoplastique.

14. Ensemble couche de support-électrode-conducteur comprenant un ensemble électrode-conducteur selon l'une des revendications 10 à 13,
**caractérisé en ce que**
l'électrode (1) est reliée à une couche de support (11) .

15. Article vestimentaire comprenant un ensemble électrode-conducteur selon l'une des revendications 10 à 13.

16. Bandage comprenant un ensemble électrode-conducteur selon l'une des revendications 10 à 13.

17. Ceinture comprenant un ensemble électrode-conducteur selon l'une des revendications 10 à 13.
